# EUROPEAN PATENT APPLICATION

(11) **EP 4 570 235 A1**
(43) Date of publication of application: **18.06.2025**
(21) Application number: 23216082.0
(22) Date of filing: 12.12.2023
(51) Int. Cl.: A61K 8/25, A61K 8/26, A61Q 11/00

(54) **DENTIFRICE COMPRISING SPHERICAL SILICA AND PERLITE PARTICLES**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The invention relates to a dentifrice composition with superior cleaning properties. The properties derive from a novel abrasive system.

## Description

### Field of Invention

The invention relates to the field of dentifrice compositions. In particular to improving the cleaning power of dentifrice compositions.

### Background to the invention

Insoluble ingredients known generally as abrasives are added to dentifrices to help with the actual removal of stains, plaque, and food particles. These components give the toothpaste the grit required to lift stubborn materials from the tooth surface.

The two abrasives that are most frequently used in toothpastes are silica and calcium carbonate. The level of abrasion (and therefore the amount of abrasive added) of the dentifrice is always a careful balancing act. A high level of abrasion will clean the tooth surface effectively, but also risks causing excessive damage to the tooth enamel itself.

The designers of dentifrice are always looking to optimise this balance between improving cleaning power and tooth enamel protection.

### Statements of the invention

In its broadest aspect the invention comprises a dentifrice composition comprising spherical silica particles and perlite particles.

In a further embodiment the spherical silica particles comprise between about 0.1 % and about 4 % by weight, and perlite particles comprise between about 0.1 % by weight and about 5.0 % by weight of the dentifrice composition.

In a further embodiment the spherical silica particles comprise between about 0.2 and 1.0 % by weight and the perlite particles between about 0.25 % by weight and about 2.0 % by weight of the dentifrice composition.

In a further embodiment the spherical silica particles have the following properties:
a. a pore volume from 0.03 mL/g to less than 0.1 mL/g
b. a mean particle size from 1 µm to 10 µm;
c. a BET surface area of 50m²/g or less;
d. an oil absorption capacity of 20 to 50 mL/100 g; and
e. a water content of less than 0.2 % wt %.

In a further embodiment the perlite particles have a D50 particle size of less than 50 µm

In a further embodiment the spherical silica particles comprise between 0.25 % and 1.0 % by weight and the perlite particles comprise between 1.25 % and 1.75 % by weight of the dentifrice composition. In a further embodiment the composition further comprises at least one surfactant and at least one humectant.

In a further embodiment the dentifrice composition further comprises a source of fluoride.

In a further embodiment the dentifrice composition comprises a further abrasive.

In a further embodiment the dentifrice composition comprises an anti-sensitivity agent

In a further embodiment the dentifrice composition comprises water.

### Detailed description of the invention

As used herein, the term "non-aqueous" means anhydrous or substantially free of water. The individual components of the dentifrice composition may contain limited amounts of water as long as the overall composition remains substantially free of water.

As used herein the term "dentifrice" includes any semi-solid preparation in the form of a paste, cream or gel for use in cleaning all or a portion of the oral cavity of an individual.

As used herein the term "oral cavity" means an individual's teeth and gums including all periodontal regions including teeth down to the gingival margins and/or the periodontal pockets.

A combination of perlite and spherical silica has been found to form a highly effective abrasive system for dentifrice compositions.

The combination specific combination gives rise to a dentifrice with superb cleaning power over other known abrasives (High PCR scores), combined with even less wear caused to tooth enamel (Low RDA scores)

Perlite is an amorphous volcanic glass that has a relatively high water content, typically formed by the hydration of obsidian. It occurs naturally and has the unusual property of greatly expanding when heated sufficiently. It is a commercial product useful for its low density after processing.

Perlite has also been shown to make an effective dentifrice abrasive, see J Clin Dent2013;24(3):88-93.

A non-limiting commercial example of perlite particles that are suitable for use in the present invention include, ImerCare^{®} PerlWhite 19 supplied by Imerys Filtration EMEA.

Preferably the perlite particles of the present invention have a pH (measured as a 10% slurry in water) between 6-8.

Preferably the perlite of the present invention have a BET surface area of at least 1.0 m²/g more preferably at least 1.25 m²/g and most preferably at least 1.50 m²/g.

Preferably the oil absorption of the perlite particles of the present invention is at least about 100 %, preferably at least about 120 % and most preferably at least about 140 % by weight of the perlite.

Preferably the perlite comprises between about 0.75 % and about 2.5 %, more preferably between about 1.0 % and about 2.0 % and most preferably about 1.5 % by weight.

Preferably the perlite particles have a d50 particle size of less than 50 µm, more preferably a d50 particle size of less than 30 µm, more preferably a d50 particle size of less than 20 µm.

Spherical silica has been shown to have good properties for dentifrice abrasives. See US 11,246,809 which is herein included by reference.

Non limiting examples of spherical silica that are suitable for use in the invention include NP-30^{®} by Sunsphere and MFIL-GS^{®} silica by Madhu PVT. Ltd.

Preferably the dentifrice compositions of the present invention comprise spherical silica particles between about 0.1 % and about 4 % by weight, and perlite between about 0.1 % by weight and about 5.0 % by weight of the dentifrice composition.

Preferably the spherical silica comprises between about 0.2 % and about 2.0 %, more preferably between about 0.25 % and 1.0 % by weight of the dentifrice composition.

Preferably the spherical silica particles have the following properties
a. a pore volume from 0.03 mL/g to less than 0.1 mL/g
b. a mean particle size from 1 µm to 10 µm;
c. a BET surface area of 50 m²/g or less;
d. an oil absorption capacity of 20 to 50 mL/100 g; and
e. a water content of less than 0.2 % wt %.

### Other dentifrice ingredients

Dentifrice compositions of the present invention may be aqueous or non- aqueous.

A non-aqueous carrier useful in the present invention typically comprises a thickening agent and one or more formulation solvent(s). Optionally, a further dentally acceptable abrasive may be included in the non-aqueous carrier.

Advantageously, a thickening agent may be present in the formulation to give the product a rheology closer to that of a conventional dentifrice. Suitably the thickening agent comprises a carboxyvinyl polymer such as a carbomer. A carbomer comprises synthetic high molecular-weight cross-linked polymers of acrylic acid. The polymer chains formed of repeating units of acrylic acid may be cross-linked with, for example: allyl sucrose to provide a carbomer available commercially in one form as Carbopol^{™} 934; ethers of pentaerythritol to provide a carbomer available commercially in one form as Carbopol^{™} 974; or with divinyl glycol, available commercially in one form as Noveon^{™} AA-1. CarbopolTM polymers are manufactured by B.F. Goodrich Company.

In one embodiment the carboxyvinyl polymer comprises CarbopolTM 974. The carboxyvinyl polymer may be present in the range of from about 0.1 to about 7.5% by weight of the dentifrice composition. In one embodiment the carboxyvinyl polymer is present in an amount from about 0.3 to about 1.0% by weight of the dentifrice composition.

The dentifrice composition according to the invention may contain at least one surfactant. The composition may comprise two or more surfactants.

The total amount of surfactant in the dentifrices of the present invention may comprise between about 0.1 % and about 15 % by weight, preferably between about 0.5 % and about 10 % by weight and most preferably between about 1.0 % and about 5.0 % by weight of the dentifrice composition. The surfactants may comprise any known for use in the art for oral care use. The skilled person will be aware of many possible suitable surfactants.

A composition according to the invention comprises may comprise a surfactant system. A surfactant system may comprise a first surfactant and a second surfactant. In certain embodiments the surfactant system consists of a first surfactant and a second surfactant wherein the second surfactant consists of a mixture of surfactants.

A suitable first surfactant belongs to the class of compounds known as betaines. Structurally, betaine compounds contain an anionic functional group such as a carboxylate functional group and a cationic functional group such as quaternary nitrogen functional group separated by a methylene moiety. They include n-alkyl betaines such as cetyl betaine and behenyl betaine, and n-alkylamido betaines such as cocoamidopropyl betaine. In one embodiment the betaine is cocoamidopropyl betaine, commercially available under the trade name Tego Betain. Suitably the betaine is present in an amount ranging from about 0.05 to about 4% by weight of the dentifrice composition, for example from about 0.2 to about 2.0% by weight of the dentifrice composition.

A second surfactant for use in the surfactant system of a composition according to the invention is selected from a taurate or a C10-20 alkyl sulphate surfactant. Taurate surfactants useful in the present invention are salts of fatty acid amides of N-methyl taurine. They conform generally to the structural formula:

RC(O)N(CH₃)CH₂CH₂SO₃M

Where RC(O)- represents a fatty acid radical and M represents sodium, potassium, ammonium or triethanolamine. Fatty acids having carbon chain lengths of from 10 to 20, including those derived from coconut, palm and tall oil are used. In one embodiment the fatty acid is derived from coconut. In one embodiment, sodium salts are used. In one embodiment the taurate is sodium methyl cocyl taurate. This taurate surfactant is sold under the trademark by Adinol CT by Croda.

The taurate surfactant may be present in an amount from about 0.1 % to about 10 % of the dentifrice composition. In one embodiment the taurate surfactant is present in an amount from about 0.1 % to about 5 % by weight of the non aqueous composition. In one embodiment the taurate surfactant is present in an amount from about 0.5 to about 2.0% by weight of the dentifrice composition.

Alkyl sulphate surfactants of use in the invention have the following structural formula:

R¹OSO₃M

R¹ represents a fatty alcohol moiety and M represents sodium, potassium, ammonium or triethanolamine. Fatty alcohols having carbon chain lengths of from about 10 to about 20, including those derived from coconut, palm oil and tall oil. In one embodiment, the fatty alcohol is lauryl alcohol. In one embodiment, a sodium salt is used. In one embodiment the alkyl sulphate is sodium lauryl sulphate.

The alkyl sulphate surfactant may be present in an amount from about 0.1 % to about 10% of the dentifrice composition. In one embodiment the alkyl sulphate surfactant may be present in an amount from about 0.1 % to about 5 % by weight of the dentifrice composition. In one embodiment the alkyl sulphate surfactant is present in an amount from about 0.5 % to about 2.0 % by weight of the dentifrice composition.

In certain embodiments, the surfactant system consists of a first surfactant which is a betaine, and a second surfactant which consists of a mixture of a taurate and a C10-20 alkyl sulphate surfactant as hereinabove described. In one embodiment the surfactant system consists of a first surfactant which is a betaine and second surfactant which consists of a mixture of sodium methyl cocyl taurate and sodium lauryl sulphate.

In one aspect a composition according to the invention comprises a dentifrice additive that is unstable or incompatible with an aqueous environment. An example of such an additive is a bioactive glass of the type disclosed in WO96/10985, WO 97/27158 and WO 99/13852.

In one embodiment the bioactive glass for use in the invention has a composition consisting of about 45 % by weight silicon dioxide, about 24.5 % by weight sodium oxide, about 6 % by weight phosphorus oxide, and about 24.5 % by weight calcium oxide. One such bioactive glass is available commercially under the trade name, NovaMin^{®}, also known as 45S5 Bioglass^{®}.

The bioactive glass is present in an amount ranging from about 1 to about 20% by weight of the dentifrice composition. In one embodiment, the bioactive glass is present in an amount from about 1 to about 15% by weight of the dentifrice composition. In an alternative embodiment, the bioactive glass in the dentifrice composition is present in an amount from about 1 % to about 10% by weight of the dentifrice composition. In a further alternative embodiment, the bioactive glass is present in an amount from about 2 % to about 8 % by weight of the dentifrice composition.

Suitably a dentifrice composition according to the invention may further comprise an inorganic thickening agent such as a thickening silica. Suitably, the thickening agent is a thickening silica, for example, a colloidal hydrated silica, available commercially for example as Sident 22S or Syloid 244FP.

In one possible embodiment the thickening silica is present in the range of from about 0 to about 15%, suitably from about 5.0 to about 15.0% by weight of the dentifrice composition.

Suitable solvents for use in the present invention include glycerin, sorbitol, propylene glycol, polyethylene glycol or mixtures thereof. In one embodiment the solvent comprises glycerin. It is well known that commercially available glycerin may contain between 0.1-2.0 % by weight of water which is in association with the glycerin. Typically, this amount is < 0.5 % for example between 0.1-0.5 % by weight of the glycerin. This small amount of water is bound to the glycerin and is therefore not available to the other ingredients. The skilled person would still consider a composition containing glycerin as being non-aqueous.

In one embodiment the solvent comprises polyethylene glycol. Suitably, the polyethylene glycol will be selected from PEG 300, PEG 400 and mixtures thereof. In one embodiment the polyethylene glycol comprises PEG 400.

In one embodiment the solvent comprises a mixture of glycerin and polyethylene glycol.

The formulation solvent is used to make the formulation up to 100%, and suitably the total amount of solvent may be present in the range of from about 20 to about 95% by weight of the dentifrice composition.

Suitably the solvent comprises glycerin present from about 35 % to about 75% by weight. In one embodiment the glycerin is present from about 50 % to about 70% by weight of the dentifrice composition.

Suitably the solvent comprises polyethylene glycol present from about 0.1% to about 40% by weight of the dentifrice composition. In one embodiment the polyethylene glycol is present from about 15 % to about 25 % by weight of the dentifrice composition.

In order to produce a composition that is smooth and does not show any signs of stickiness, use of a particular ratio of carboxyvinyl polymer to polyethylene glycol is desirable.

Advantageously, the ratio of carboxyvinyl polymer to polyethylene glycol is in the range of about 1:15 to about 1:30.

The dentifrice compositions of the present invention should not require additional abrasives.

However additionally dentally acceptable abrasive may optionally be added to the dentifrice composition. Suitable abrasives for use in the dentifrice composition include, for example, amorphous, gelled, precipitated or fumed silica, zinc orthophosphate, sodium bicarbonate (baking soda), plastic particles,, calcium carbonate, calcium pyrophosphate, insoluble metaphosphates or mixtures thereof.

The silica abrasive may be a natural amorphous silica, for instance diatomaceous earth; or a synthetic amorphous silica such as a precipitated silica. By way of example, silica abrasives include those marketed under the following trade names Zeodent, Sident, Sorbosil or Tixosil by Huber, Degussa, Ineos and Rhodia respectively.

Suitably a silica abrasive is present in an amount up to 25% by weight of the total composition, for example from 2 to 20% by weight for example from 5 to 15% by weight of the total composition.

Generally, an amount of abrasive suitable for use in the non-aqueous composition of the present invention will be empirically determined to provide an acceptable level of cleaning and polishing, in accordance with the techniques well known in the art.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25 to 3500pm of fluoride ions, preferably from 100 to 1500ppm.

Polyphosphates are known to help retard calculus formation and are examples of anticalculus agents suitable for use in the invention. A polyphosphate is generally understood to consist of two or more phosphate groups arranged primarily in a linear configuration, although some cyclic derivatives may be present. Polyphosphates of use in the invention include pyrophosphates, polyphosphates having three or more polyphosphate groups such as sodium tripolyphosphate, and polyphosphates having four or more polyphosphate groups such as tetrapolyphosphate and hexametaphosphate among others.

Compositions of the invention may further comprise an anti erosion agent, for example a polymeric mineral surface-active agent as described in WO 04/054529 (Procter & Gamble).

Compositions of the present invention will contain additional formulating agents such as flavouring agents, sweetening agents, opacifying or colouring agents and preservatives, selected from those conventionally used in an oral hygiene composition art for such purposes.

In general, the optional agents may be used in a minor amount or proportion of the overall formulation. By way of example, such components are usually present in from about 0.001 to about 5% by weight of the dentifrice composition.

The dentifrice composition typically has a viscosity suitable for application to the oral cavity. The viscosity will vary depending on the type of dentifrice composition made and the ultimate use thereof. One of skill in the art can readily prepare compositions with suitable viscosities for use in the oral cavity from the teachings provided herein.

The compositions according to the present invention may be prepared by admixing the ingredients in the appropriate relative amounts in any order that is convenient.

The invention is further illustrated by the following Examples.

### Results

To demonstrate the effectiveness of the novel abrasive system a comparison with a typical commercial paste with a known silica abrasive was carried out.

The tested formulas are on table 1.

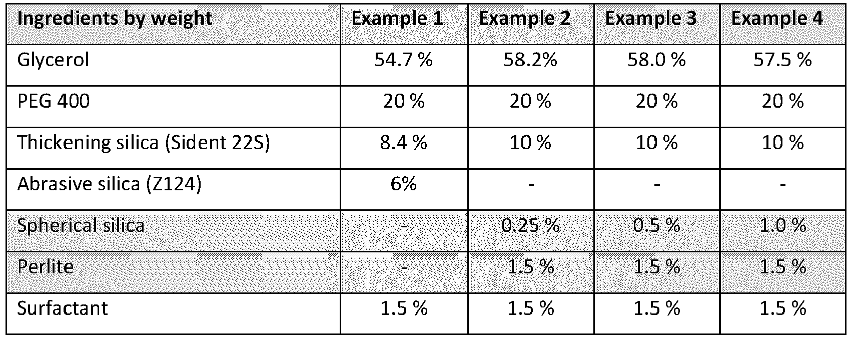

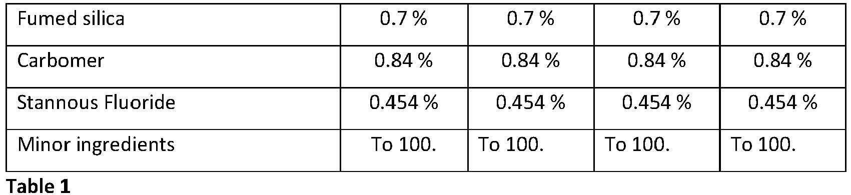

Example 1 is a commercial Sensodyne formulation.

Examples 2, 3 and 4 were modified versions of that commercial product with the commercial silica-based abrasive removed and replaced with the abrasive systems of the present invention.

The four compositions were tested by known standard methods to compare their cleaning power and abrasion of the tooth surface. The results shown on table 2.

The three examples of the invention showed increased cleaning power over the commercial product in the Pellicle cleaning ratio test (PCR).

All three test samples also showed a dramatic drop in tooth wear over the commercial product in the radioactive dentine analysis test.

**Table 2**

| **Formula** | **PCR** | **RDA** |
|---|---|---|
| Example 1 (commercial) | 133.2 | 120 |
| Example 2 | 149.4 | 52.9 |
| Example 3 | 168.2 | 59.8 |
| Example 4 | 175.4 | 61.9 |

Thus the abrasive system of the present invention is clearly superior to the known silica systems in use currently.

### Testing methods

### Pellicle Cleaning Ratio Test (PCR) as a Measure of Stained Pellicle Removal from Bovine Enamel Introduction

Previous studies (J. Dent. Res., 61:1236, 1982) have indicated that the results of the *in-vitro* PCR test with dentifrice slurries may be considered to be predictive of clinical findings with a reasonable degree of confidence.

Dentifrice formulations were assessed for their ability to remove pellicle stain from bovine enamel and compared with similar comparative formulations (not encompassed within the scope of the invention) containing alternative silica abrasive materials.

### Methodology

Bovine, permanent, central incisors were cut to obtain labial enamel specimens approximately 8×8mm². The enamel specimens were then embedded in an autopolymerizing methacrylate resin so that only the enamel surfaces were exposed. The enamel surfaces were then smoothed on a lapidary wheel and polished with flour of pumice and water, then sonicated to remove excess debris. They were then lightly etched (60 seconds in 0.12M HCl, 30 seconds in saturated NaCO₃ and 60 seconds in 1.0% phytic acid) to expedite stain accumulation and adherence. They were then placed on a rotating rod alternately exposing them to staining broth containing gastric mucin as a protein source and coffee, tea and FeCl₃ 6H₂O as staining sources (i.e., 1.35g coffee, 1.35g tea, 0.02g Fe and 1.0 g mucin in 400ml) for a minimum of 10 days to ensure the specimens developed sufficient stain.

The amount of in vitro stain was graded photometrically (Minolta 2600d, colorimeter) using only the L value of the LAB scale. The area of the specimens measured was a %inch diameter circle in the centre of the enamel sample. Specimens with L value measurements between 30-38 (30 being more darkly stained) were used. On the basis of these measurements, the specimens were divided into groups of 16 specimens each, with each group having the same average baseline measurement.

The specimens were then mounted on a mechanical V-8 cross-brushing machine equipped with soft nylon-filament (Oral-B 40 Indicator) toothbrushes. Toothbrushes were conditioned by running the brushing machine for 1,000 strokes in deionised water. Tension on the enamel surface was adjusted to 150g. The dentifrices were used as slurries prepared by mixing 25g of dentifrice with 40ml of deionised water. The ADA reference material was prepared by mixing 10g of material and 50ml of a 0.5% CMC solution. The specimens were brushed for 800 double strokes. To minimise mechanical variables, one specimen per group was brushed on each of the eight brushing heads. Fresh slurries were used for each specimen brushed. Following brushing, specimens were rinsed, blotted dry, and measured again for stain as previously described.

The difference between the pre- and post-brushing stain measurements was determined and the mean and standard error calculated for the reference group. The cleaning ratio for the reference material group was assigned a value of 100. The mean decrement for the reference group was divided into 100 to obtain a constant value to multiple times each individual test decrement within the study. The individual cleaning ratio of each specimen was then calculated (decrement X constant). The mean and standard error mean (SEM) for each group (N=16) was then calculated using the individual cleaning ratios. The larger the value of the cleaning ratio, the greater the amount of stained pellicle removed.

Statistical analyses of the individual means were performed with a one-way analysis of variance (ANOVA) model using Sigma Stat (3.1) Software. Since the ANOVA indicates significant differences, the individual means were analysed by the Student Newman-Keuls (SNK) test.

### RDA: Relative Dentine Abrasivity

The most industry standard way of measuring how abrasive a toothpaste is. This procedure is described in ISO 11609 and ANSI/ADA Standard No. 130.

Each test is a result that is relative to the control and the lower the better.

Dentifrice formulations were assessed for their abrasivity (as determined by RDA) and compared as against similar comparative formulations

### Methodology

The procedure used was the ISO/ADA recommended procedure for determination of dentifrice abrasivity. The dentin specimens (8) were placed in a neutron flux under the controlled conditions outlined by the ISO/ADA. The specimens were then mounted in methyl methacrylate so they would fit in a V-8 cross-brushing machine. The specimens were brushed for 1,500 strokes in a precondition run (since the teeth had been used previously) using a slurry consisting of 10g ISO/ADA reference material in 50ml of a 0.5% CMC glycerin solution. The brushes used were those specified by the ISO/ADA and had been used in a previous study. The brush tension was 150g. Following the precondition run, the test was performed using 150g and 1,500 strokes in a sandwich design in which each test material slurry (25g dentifrice/40ml water) was flanked by reference material slurries (10g ADA reference/50 ml 0.5% CMC).

A 1ml sample was removed from each reference material slurry, weighed (0.01g) and added to 4.5ml of scintillation cocktail. The samples were well mixed and immediately placed on the scintillation counter for radiation detection. Following counting, the net counts per minute (CPM) values were divided by the weight of the sample to calculate the net CPM/gram of slurry. The net CPM/g of the pre- and post-ADA reference material for each test slurry were then calculated and averaged to use in the calculation of the RDA for the test material. The ISO/ADA material was assigned a value of 100 and its ratio to the test material was calculated.

Statistical analyses were performed by a one-way ANOVA model using Sigma Stat Software (13.0). Since significant differences were indicated, the individual means were analysed by the Student Newman Keuls (SNK) test.

## Claims

1. A dentifrice composition comprising spherical silica particles and perlite particles.

2. The dentifrice composition according to claim 1 wherein the spherical silica particles comprise between about 0.1 % and about 4.0 % by weight, and perlite particles between about 0.1 % by weight and about 5.0 % by weight.

3. The dentifrice composition according to either claim 1 or claim 2 wherein the spherical silica particles comprise between about 0.2 % and 1.0 % by weight and the perlite particles between about 0.25 % by weight and about 2.0 % by weight.

4. The dentifrice composition according any of the previous claims wherein the spherical silica particles have the following properties:
a. a pore volume from about 0.03 mL/g to less than about 0.1 mL/g
b. a mean particle size from about 1 µm to about 10 µm;
c. a BET surface area of about 50 m²/g or less;
d. an oil absorption capacity of about 20 to about 50 mL/100 g; and
e. a water content of less than about 0.2 % by weight.

5. The dentifrice composition according to any of the previous claims wherein the perlite particles have a d50 particle size of less than 50 µm

6. The dentifrice composition according to any of the previous claims wherein spherical silica particles comprise between about 0.25 % and about 1.0 % by weight and the perlite particles comprises between about 0.75 % and about 1.5 % by weight.

7. The dentifrice composition according to any of the previous claims wherein the composition further comprises at least one surfactant, and at least one humectant.

8. The dentifrice composition according to any of the previous claims wherein the composition further comprises a source of fluoride.

9. The dentifrice composition according to any of the previous claims wherein the composition comprises a further abrasive.

10. The dentifrice composition according to any of the previous claims wherein the composition comprises an anti-sensitivity agent.

11. The dentifrice composition according to any of the previous claims wherein the composition comprises water.
